# EUROPEAN PATENT APPLICATION

(11) **EP 3 254 707 A1**
(43) Date of publication of application: **13.12.2017**
(21) Application number: 16461524.7
(22) Date of filing: 09.06.2016
(51) Int. Cl.: A61L 9/03

(54) **A WARMER FOR A SCENTED SUBSTANCE**

(71) Applicant: Korona Candles S.A., 98-300 Wielun (PL)
(72) Inventor: Wrobel, Andrzej, 98-300 Wielun (PL); Spiegowski, Grzegorz, 98-300 Wielun (PL); Zawadzka, Monika, 98-345 Mokrsko (PL)
(74) Representative: Kancelaria Eupatent.pl Sp. z.o.o

(57) **Abstract**

A warmer for a scented substance, the warmer comprising a melting cap (100) having side walls (110) and a support surface (120) formed between the side walls, wherein the support surface (120) comprises an elevated portion (122) on which said scented substance is placed and a lowered portion (124) which is positioned below the elevated portion (122) and has at least one opening (121). In use said scented substance is heated and melts so flowing from the elevated portion towards said lowered portion and here through the opening.

## Description

### TECHNICAL FIELD

The present invention relates to a warmer for a scented substance.

### BACKGROUND

There is known a scented oil warmer, which is a device for heating up a scented portion of oil with a candle flame. The candle is typically provided in form of a tealight candle. A typical scented oil warmer comprises a base for supporting the candle and a bowl for scented oil located above the candle. Other known scented substances can be used in place of scented oils, such as a scented wax melt or another scented mass. The flame of the candle located below the bowl with the scented substance, warms it up and as a result the evaporation of the substance is increased. Pleasant scents help to mask unpleasant odors of the environment and may additionally have a positive influence on human mood. It is believed that particular scents may relieve stress, reduce headaches etc.

Conventional scented oil warmers for melting scented wax melts (pressed and cast) have the form of a one piece structure. As mentioned above, the wax is warmed up and melted in the bowl by means of a tealight candle located in a bottom part of the warmer. Therefore, after the scent evaporates from the melted wax, the rest of the wax ingredients remain in the bowl as a waste and have to be eliminated before the next use, in order not to improve the aestheticism of the bowl and eliminate mixing of scents if another scent is to be used. This causes troublesome cleaning for the user.

Therefore, there is a need to provide an improved warmer for a scented substance having a construction that facilitates its use.

### SUMMARY

There is disclosed a warmer for a scented substance, the warmer comprising a melting cap having side walls and a support surface formed between the side walls, wherein the support surface comprises an elevated portion and a lowered portion which is positioned below the elevated portion and has at least one opening.

The side walls may have side openings.

The at least one opening can be located in the lowest part of the lowered portion.

The at least one opening may have a diameter smaller than 5 mm.

The outer outline of the cross-section of the melting cap may change from the top to the bottom.

The melting cap may further comprise a handle.

The elevated portion can be located at the centre of the melting cap.

The elevated portion can be located along the outer periphery of the melting cap.

The elevated portion may have flat top parallel to the bottom surface of the melting cap.

The elevated portion may have a top non-parallel to the bottom surface of the melting cap.

The warmer may further comprising a cup positioned below the melting cap. The cup may comprise a flammable mass.

The flammable mass can be scented.

The warmer may comprise a scented substance on top of the support surface.

### BRIEF DESCRIPTION OF DRAWINGS

The present invention is shown by means of example embodiments on a drawing, in which:
Fig. 1 shows a warmer for a scented substance according to the invention;
Fig. 2 shows a construction of a support surface of a melting cap in a top view;
Fig. 3 shows a cross-section of the construction of the support surface of the melting cap;
Figs. 4A-4B show another embodiment of the warmer in a general view and in a cross-section;
Figs. 5-7 show various embodiments of a cross-section of the melting cap;
Figs. 8-15 show various embodiments of the warmer.

### DETAILED DESCRIPTION

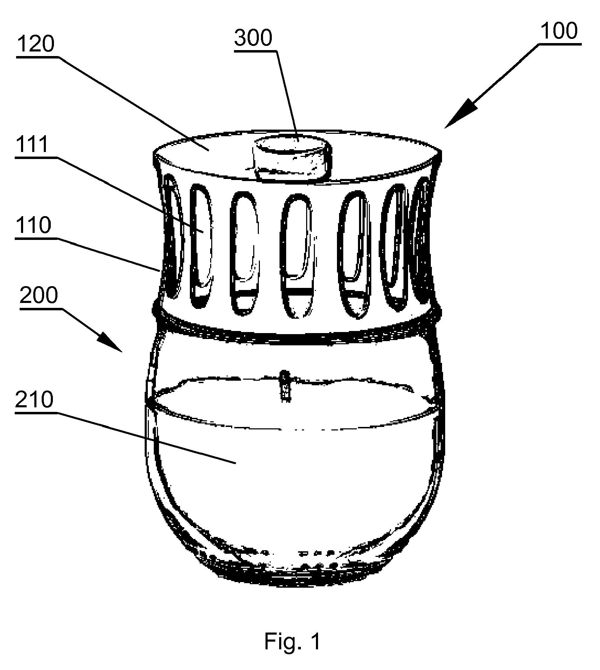

Fig. 1 shows a warmer for a scented substance according to the invention. The warmer can be considered as a self-cleaning warmer. The warmer comprises a melting cap 100, which may be positioned over a candle cup 200, in particular on the upper edge of the candle cup. Alternatively, the melting cap 100 can be used as a stand-alone warmer.

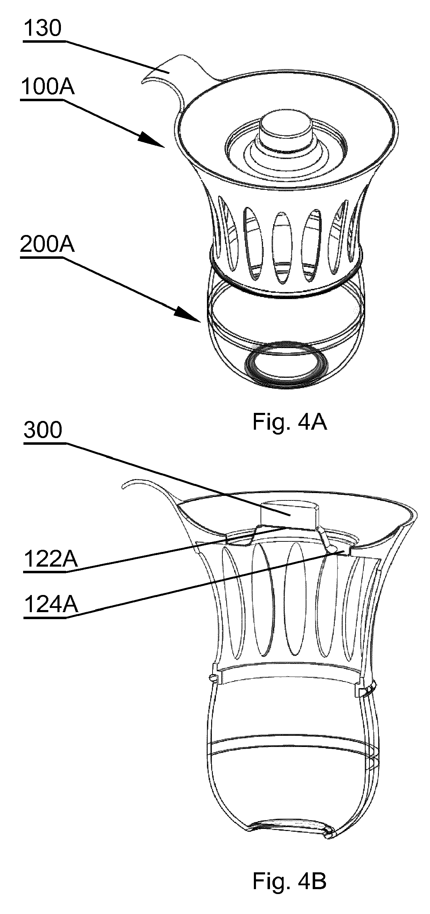

The melting cap 100 has side walls 110. For example, it may have a substantially cylindrical shape - in this example, a shape of a cylinder with a concave side wall. The term "side walls" is understood to comprise a plurality of distinct side walls or a substantially continuous single side wall, such as the cylindrical side wall. The melting cap may have a cross-section other than round, e.g. triangular, rectangular, polygonal or other shapes (for example as shown in Figs. 4-15). The side walls 110 may be adjacent to each other or separate from each other. The side walls 110 may have openings 111 to allow air to enter inside the space between the side walls and to the candle. A support surface 120 for the scented substance is formed between the side walls 110. The support surface 120 acts as a bowl 120, wherein the scented substance 300 is placed. The support surface 120 with the flammable scented mass 300 is supposed to be warmed up with a flame of a candle to be positioned below the support surface 120. As a result of warming, the scented mass melts, its evaporation is increased and the scent is released to the atmosphere surrounding the melting cap 100.

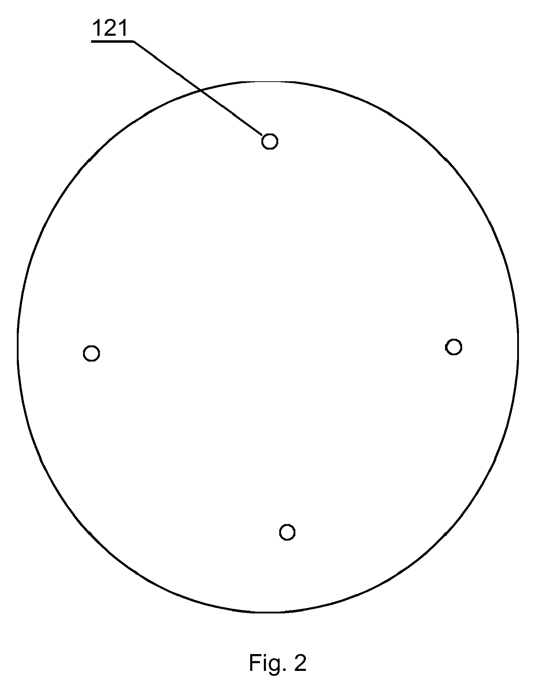

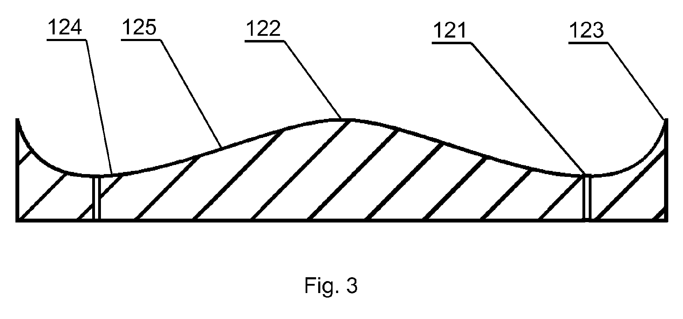

Fig. 2 shows the support surface 120 of the melting cap in a top view. The support surface 120 has at least one through opening 121 (shown in a cross-section in Fig. 3), which allow the melted scented mass 300 to flow through from the top surface of the support surface 120 to the space between the side walls 110.

If the melting cap 100 is positioned over the candle cup 200, the scented mass 300 flows on the top of the flammable mass 210 within the candle cup 200 and mixes therewith. As a result, the mixture of the scented mass is burnt along with the flammable mass of the candle cup 200, which in consequence increases the burning time of the candle and allows all the scented mass to be consumed. Therefore, the scented mass is preferably flammable. Owing to this, the amount of the flammable mass 300 that remains on the top of the support surface 120 is reduced and therefore the amount of work for the user to clean the top of the support surface 120 is at least reduced or eliminated. In case only a small portion of the scented mass 300 remains on the top of the support surface, the problem of mixing it with another scent to be used is minimized.

Fig. 3 shows a cross-section of the support surface 120 of the melting cap 100. In the central part of the support surface 120 there is an elevated portion 122, on which the flammable scented mass 300 in a solid form is supposed to be positioned before use. As a result of warming, the scented mass 300 melts and flows down the elevated portion 122 towards lowered portion 124 where the openings 121 are distributed. Preferably, the elevated portion 122 is in the center of the support surface 120 and the lowered portion 124 surrounds the elevated portion 122. In alternative embodiments, the elevated portion 122 is offset from the center, for example is located at one side of the support surface 120 and the lowered portion 124 is located at an opposite side of the support surface 120. Furthermore, the elevated portion 122 may surround the lowered portion 124. Preferably, the openings 121 are positioned in the lowest part of the lowered portion 124. The edge 123 of the support surface 120 is preferably positioned above the surface of the openings 121 so that the melted mass 300 does not flow outside the support surface 120 but only through the openings 121 to the interior of the cap. The elevated portion 122 is joined with the lowered portion 124 by preferably a smooth, sliding surface 125 via which the melted mass 300 may flow from the elevated portion 122 to the lowered portion 124.

Preferably, the elevated portion 122 has a convex cross-section and the lowered portion 124 has a concave cross-section.

The scented mass 300 may have a shape adapted to be positioned and remain still on the elevated portion 122 when the warmer is not in use. For example, the scented mass 300 may have a form of a cylinder, preferably with a concave bottom, to stay still on top of the convex elevation portion 122 as shown in Fig. 1.

The number and size of openings may be selected depending on the particular model of the warmer. There may be 1, or a few, or a dozen or so, or a few dozen, or even a few hundred openings. The openings may have a circular cross-section and a diameter below 5 mm, or below 1 mm, or below 0,5 mm, or below 0,3 mm, or below 0,2 mm, or below 0,1 mm, and preferably above 0,05 mm. However, other cross-sections of the openings can be used as well.

The flammable mass 210 of the candle 200 may also comprise a scent agent. The scent of the flammable mass 210 may be different than the scent of the scented substance 300, therefore different scents compositions may be obtained. The users can experiment on their own to join different scents.

The melting cap 100 can be made of metal, for example aluminum.

The cup 200 can be made of metal, plastic or glass.

Figs. 4A-4B show another embodiment of a warmer in a general view and in a cross-section. The melting cap 100A has a handle 130 that facilitates lifting off the melting cap from the candle cup 200A.The elevated portion 122A and the lowered poriton 124A are flat (the openings in the lowered portion are small and not visible in this scale of the drawing).

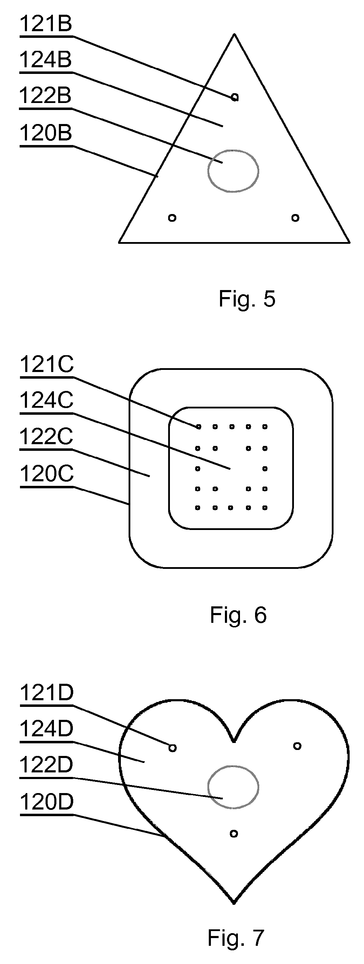

Fig. 5 shows another embodiment of a melting cap 100B in a top view, having a triangular cross-section with three holes 121 B distributed along the lowered portion 124B and a circular central elevated portion 122B.

Fig. 6 shows another embodiment of a melting cap 100C in a top view, having a rectangular cross-section with twenty holes 121C distributed at the lowered portion 124C and a circular circumferential elevated portion 122C (on which a scented mass in form of four sticks or a rectangular frame can be placed).

Fig. 7 shows another embodiment of a melting cap 100D in a top view, having a fancy cross-section (in this case: heart-shaped) with three holes 121 D distributed along the lowered portion 124D and a circular central elevated portion 122D.

The outer outline of the cross-section of the melting cap may 100 be the same or similar along the whole height of the melting cap (i.e. it may have a circular, square, triangular, polygonal or fancy shape from the top to the bottom). Alternatively, it may change from one shape at the top (e.g. square, triangular, polygonal or fancy shape) to another shape at the bottom (e.g. circular, to accommodate the melting cap on a standardized candle cup with a circular top upper edge).

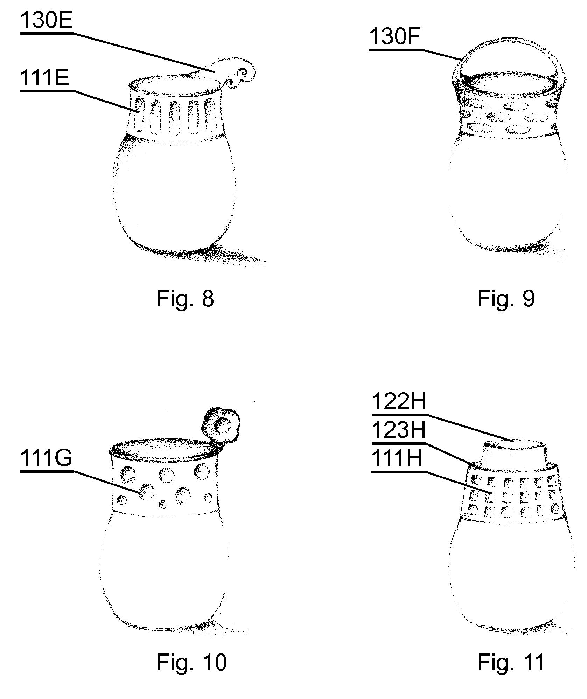

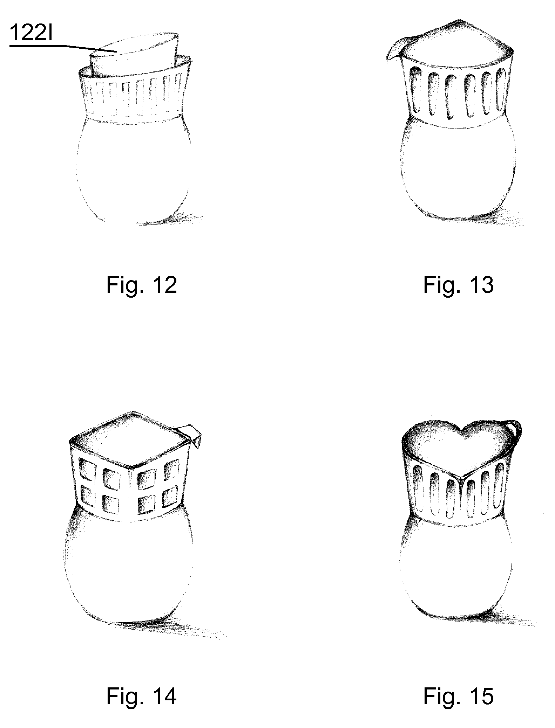

Figs. 8-15 show various embodiments of the warmer. The melting cap may have elongated vertical side holes (111 E of Fig. 8), or round side holes (111 G of Fig. 10) or square side holes (111 H of Fig. 11). The melting cap may have a side handle (130E of Fig. 8) or a top handle (130F of Fig. 9). The elevated portion 122H can be raised substantially higher than the edge 123H surrounding the lowered portion (Fig. 11). The elevated portion 122I may be non-horizontal (non-parallel to the bottom of the melting cap, Fig. 12). Figs. 13-15 show embodiments with a melting cap having a cross-section at the upper portion similar to that of Figs. 5-7.

## Claims

1. A warmer for a scented substance, the warmer comprising a melting cap (100) having side walls (110) and a support surface (120) formed between the side walls, **characterized in that** the support surface (120) comprises an elevated portion (122) and a lowered portion (124) which is positioned below the elevated portion (122) and has at least one opening (121).

2. The warmer according to claim 1, wherein the side walls (110) have side openings (111).

3. The warmer according to any of previous claims, wherein the at least one opening (121) is located in the lowest part of the lowered portion (124).

4. The warmer according to any of previous claims, wherein the at least one opening (121) has a diameter smaller than 5 mm.

5. The warmer according to any of previous claims, wherein the outer outline of the cross-section of the melting cap (100) changes from the top to the bottom.

6. The warmer according to any of previous claims, wherein the melting cap (100) further comprises a handle (130).

7. The warmer according to any of previous claims, wherein the elevated portion (122) is located at the centre of the melting cap (100).

8. The warmer according to any of claims 1-6, wherein the elevated portion (122) is located along the outer periphery of the melting cap (100).

9. The warmer according to any of previous claims, wherein the elevated portion (122) has a flat top parallel to the bottom surface of the melting cap (100).

10. The warmer according to any of previous claims, wherein the elevated portion (122) has a top non-parallel to the bottom surface of the melting cap (100).

11. The warmer according to any of previous claims, further comprising a cup (200) positioned below the melting cap (100).

12. The warmer according to claim 11, wherein cup (200) comprises a flammable mass (210).

13. The warmer according to claim 12, wherein the flammable mass (210) is scented.

14. The warmer according to any of previous claims, further comprising a scented substance on top of the support surface (120).
